(19) Europäisches Patentamt
European Patent Office
Office européen des brevets

(11) Veröffentlichungsnummer : **0 056 154**
**B1**

(12) **EUROPÄISCHE PATENTSCHRIFT**

(45) Veröffentlichungstag der Patentschrift : **23.05.84**

(51) Int. Cl.³ : **C 07 C 69/743**, C 07 C 61/35, C 07 C 49/16, C 07 C 45/69, C 07 C 51/00, C 07 C 67/00

(21) Anmeldenummer : **81110807.5**

(22) Anmeldetag : **29.12.81**

(54) **Verfahren zur Herstellung von 2,2-Dimethyl-3-vinyl-cyclopropancarbonsäuren und deren Estern.**

(30) Priorität : **08.01.81 DE 3100354**

(43) Veröffentlichungstag der Anmeldung : **21.07.82 Patentblatt 82/29**

(45) Bekanntmachung des Hinweises auf die Patenterteilung : **23.05.84 Patentblatt 84/21**

(84) Benannte Vertragsstaaten : **BE CH DE FR GB IT LI NL**

(56) Entgegenhaltungen :
**EP-A- 0 008 340**
**DE-A- 2 621 835**
**DE-B- 2 659 973**
**HOUBEN-WEYL "Methoden der organischen Chemie", 4. Auflage, Band VIII 1952, GEORG THIEME VERLAG, Stuttgart Seiten 458 bis 462**

(73) Patentinhaber : **BAYER AG**
**Konzernverwaltung RP Patentabteilung**
**D-5090 Leverkusen 1 Bayerwerk (DE)**

(72) Erfinder : **Jautelat, Manfred, Dr.**
**Müllersbaum 28**
**D-5093 Burscheid (DE)**
Erfinder : **Arlt, Dieter, Prof. Dr.**
**Rybniker Strasse 2**
**D-5000 Koeln 80 (DE)**

**EP 0 056 154 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von bekannten 2,2-Dimethyl-3-vinyl-cyclopropancarbonsäuren und deren Estern.

2,2-Dimethyl-3-vinyl-cyclopropancarbonsäureester und ihre Herstellung sind bekannt.

Unter den zahlreichen Synthesen zeichnen sich die Herstellungsverfahren über eine Umlagerung von α-Halogencyclobutanonen (DOS 2 638 356 und EP 2.207) durch gute Ausbeuten und einfache Durchführung aus. Die Herstellung der α-Halogencyclobutanone muß jedoch über eine mehrstufige Synthese erfolgen. Die dabei erhältlichen Ausbeuten sind nicht immer voll befriedigend.

Es wurde gefunden, daß man die bekannten 2,2-Dimethyl-3-vinyl-cyclopropancarbonsäure-Derivate der Formel I

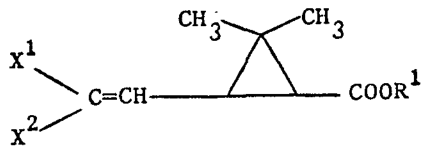
(I)

in welcher

$R^1$ für Wasserstoff, Alkyl oder den Rest eines bei Pyrethroiden verwendbaren Alkohols steht und

$X^1$ und $X^2$ gleich oder verschieden sind und für Halogen oder fluorsubstituiertes Alkyl stehen erhält, indem man

a) Polyhalogenalkane der Formel II

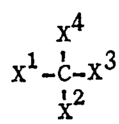
(II)

in welcher

$X^1$ und $X^2$ für die oben genannten Reste und

$X^3$ und $X^4$ gleich oder verschieden sind und für Halogen stehen an 1-Chlor-3,3-dimethyl-pent-4-en-2-on der Formel III

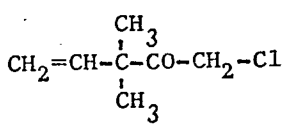
(III)

in Gegenwart von Katalysatoren, die freie Radiale liefern, oder in Gegenwart von Metallsalzen der VIII Hauptgruppe und der Nebengruppe IVa, VIIa und Ib des Periodensystems addiert und

b) die dabei erhältlichen Verbindungen der Formeln IV und V

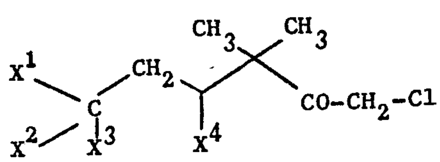
(IV)

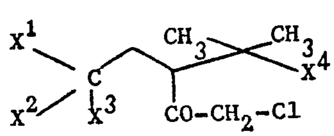
(V)

in welcher

$X^1$, $X^2$, $X^3$ und $X^4$ für die oben genannten Reste stehen einzeln oder im Gemisch mit Basen der Formel VI

$$(R^1 - O^{\ominus})_n M^{n+} \quad (VI)$$

in welcher

$R^1$ für die oben genannten Reste,

M für Alkali- oder Erdalkalimetall und

n für 1 oder 2 stehen, umsetzt.

Es ist als ausgesprochen überraschend zu bezeichnen, daß die radikalische Addition von Polyhalogenalkanen der Formel II an das ungesättigte Keton der Formel III neben den direkten Additionsprodukten der Formel IV unter Umlagerung zu isomeren Produkten der Formel V führt. Nach dem Stand der Technick (Houben-Weyl, Methoden der Org. Chemie, V/3 S. 971) war mit einer Umlagerung unter diesen Bedingungen nicht zu rechnen. Noch überraschender aber ist, daß sowohl jedes dieser isomeren Addukte IV und V als auch deren Gemisch bei der Behandlung mit Alkoholaten oder Hydroxiden ein cis-trans-Gemisch der 2,2-Dimethyl-3-vinyl-cyclopropancarbonsäuren der Formel I ergibt.

Das erfindungsgemäße Verfahren weist eine Reihe von Vorteilen auf. So erhält man bei der Einwirkung von Basen auf die Verbindungen IV und V direkt die Vinylcyclopropancarbonsäuren der Formel I in guten Ausbeuten und Reinheiten ohne, daß eventuell dabei auftretende Zwischenstufen isoliert werden müssen.

Ferner bedarf es keiner Trennung der Verbindungen IV und V, sondern sie können als Gemisch verwendet werden. Auch das Ausgangsmaterial der Formel III, 1-Chlor-3,3-dimethyl-pent-4-en-2-on, kann aus preiswerten, technisch leicht zugänglichen Stoffen, Isopren und Vinylidenchlorid, einfach hergestellt werden.

Verwendet man beispielsweise Tetrachlormethan als Ausgangsstoff, so kann der Reaktionsablauf durch das folgende Formelschema wiedergegeben werden :

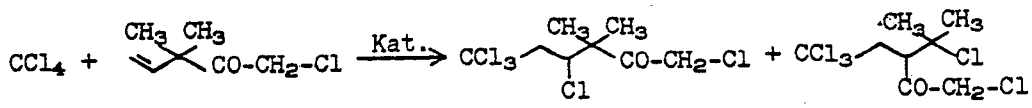


III IV V

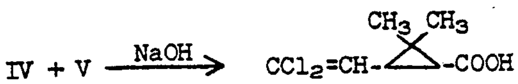


Polyhalogenalkane als Ausgangsstoffe sind durch die Formel II allgemein definiert. In der Formel II stehen $X^1$ und $X^2$, die gleich oder verschieden sein können, vorzugsweise für Chlor, Brom, Fluor und perfluorierte Alkylreste mit 1-3 C-Atomen. $X^3$ und $X^4$, die ebenfalls gleich oder verschieden sein können, stehen vorzugsweise für Chlor und Brom.

Als Beispiele für die erfindungsgemäßen verwendbaren Polyhalogenalkane seien im einzelnen genannt : Tetrachlormethan, Tetrabrommethan, Chlortribrommethan, Dichlordifluormethan, Bromchlordifluormethan, Jodtrichlormethan, 1,1,1-Trichlor-trifluorethan, 1,1,1-Tribromtrifluorethan, 1,1-Dibrom-tetrafluorethan, 2,2-Dibrom-1,1,1,3,3,3-hexafluorpropan, 1,1,1,3-Tetrachlortetrafluorpropan, 1,1,1-Trichlorpentafluorpropan.

Die Verbindung der Formel III kann in einfacher Weise nach folgendem Verfahren hergestellt werden.

Die Chlorwasserstoffaddition an Isopren führt zum Dichlorisopentan, das an Vinylidenchlorid addiert und mit Basen zum 1,1-Dichlor-3,3-dimethyl-1,4-pentadien eliminiert wird. Umsetzung mit Natriumphenolat und Hydrolyse liefern das Chlormethylketon der Formel III :

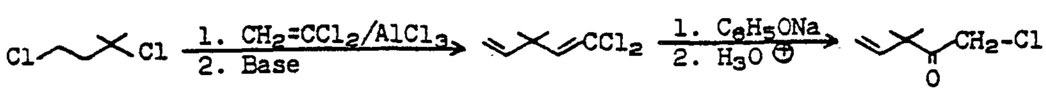


(III)

Die Addition gemäß Verfahrensschritt a) wird bei Normaldruck, im Falle von niedersiedenden Verbindungen der Formel II vorzugsweise unter Druck durchgeführt.

Der Druckbereich kann in weiten Grenzen schwanken, etwa zwischen 1 und 30 bar, bevorzugt zwischen 3 und 15 bar. Der Überdruck kann durch Aufpressen eines Inertgases, wie beispielsweise Stickstoff, erreicht werden.

Die Umsetzungstemperatur kann zwischen 50 und 200 °C liegen, bevorzugt wird jedoch zwischen 80 und 150 °C gearbeitet. Den beiden Ausgangsstoffen der Formeln II und III wird gegebenenfalls ein Verdünnungsmittel zugesetzt.

Als Verdünnungsmittel kommen in Frage : aliphatische oder aromatische Kohlenwasserstoffe, wie Benzin oder Toluol, Alkohole, wie Methanol, Ethanol, Propanol oder tert. Butanol, Nitrile wie Acetonitril oder Propionitril, DMF. Bevorzugt sind Alkohole, Acetonitril und DMF.

Zur Durchführung der Reaktion sind Katalysatoren erforderlich. Und zwar entweder

a) freie Radikale liefernde Katalysatoren, wie z. B. Azobisisobutyronitril, Benzoylperoxid oder Ditert.-butylperoxid oder

b) Metallsalze der VIII. Hauptgruppe und der Nebengruppe VIa, VIIa und Ib des periodischen Systems. Als Beispiele seien genannt : Kupfer(II)oxid, Eisen(III)oxid ; Cu(I)-, Cu(II)-, Fe(II)- und Fe(III)bromide und vor allem -chloride sowie die Chloride des Rutheniums, Rhodiums, Palladiums, Kobalts und Nickels ; Cu(II)sulfat, Fe(II)- und Fe(III)sulfat ; Cu(II)-nitrat und Eisen(III)nitrat ; Mangan(III)acetat, Kupfer(II)-acetat ; Kupfer(II)stearat ; Eisen(III)citrat ; Cu(I)cyanid ; Ruthenium(II)dichloro-tris-triphenylphosphin, Rhodium-tris-(triphenylphosphin)-chlorid ; Chrom- und Nickelacetylacetonat, Kupfer(II) acetylacetonat, Eisen(III)acetylacetonat, Kobalt(II)- und Kobalt(III)acetylacetonat, Mangan(II)acetylacetonat, Kupfer(II)-benzoylacetonat ; Eisencarbonyl-cyclopentadienyl-komplex ; Molybdäncarbonylcyclopentadienylkomplex, komplex, Ruthenium(II)acetatkomplex, Chrom- und Molybdänhexacarbonyl, Nickeltetracarbonyl, Eisenpentacarbonyl, Kobalt- und Mangancarbonyl oder

c) Katalysatormischungen, die aus einem der obigen Metallsalze, einem Amin und gegebenenfalls etwas Benzoin bestehen. Das Metallsalz kann auch als Hydrat vorliegen, das Amin kann auch als Salz, z. B. als Hydrochlorid vorliegen. Beispiele für Amine sind :

Dimethylamin, Diethylamin, Trimethylamin, Triethylamin-Diisopropylamin, n-Butylamin, Benzylamin, Ethanolamin, Anilin, Pyridin. Bevorzugt sind Dimetylamin, Diethylamin (als Hydrochloride), sowie n-Butylamin.

Es werden vorzugsweise mindestens 1,5 Mol, bevorzugt 2-10 Mol organisches Amin pro Mol Metallsalz eingesetzt. Das Metallsalz wird in Mengen von 0,01 bis 15 Mol-%, vorzugsweise 0,05 bis 10 Mol-% bezogen auf Verbindungen der Formel II eingesetzt.

Die Zugabe von äquimolaren Mengen Benzoin bezogen auf das Metallsalz ist oft von Vorteil.

Die Reaktionskomponenten der Formeln II und III werden äquimolar eingesetzt. Vorzugsweise wird das Polyhalogenalkan II im Überschuß oder gar als Verdünnungsmittel verwendet.

Die erhaltenen Additionsprodukte der Formel IV und V werden als Gemisch oder nach einer Trennung einzeln mit Basen der Formel VI (Verfahrensschritt b) umgesetzt.

In Formel VI, steht $R^1$ vorzugsweise für Wasserstoff, Alkyl mit 1-6 C-Atomen oder den Rest eines bei insektizid wirksamen Pyrethroiden verwendbaren Alkohols. M steht vorzugsweise für ein Alkalimetall, insbesondere Natrium und Kalium.

Im einzelnen seien als Beispiele für Basen genannt : Natriumhydroxid, Kaliumhydroxid, Natriummethylat, Natriumethylat, Natriumbutylat, Kalium-tert.-butylat und die Alkalisalze folgender Alkohole : 5-Benzyl-3-hydroxymethyl-furan, 5-Benzyl-2-hydroxymethylfuran, 5-Benzyl-3-hydroxymethyl-thiophen, 5-Phenoxy-5-hydroxymethyl-furan, 3-Hydroxy-4-methyl-5-allyl-cyclopenten(4)-1-on, N-Hydroxymethylphthalimid, N-Hydroxymethyl-3,4,5,6-tetrahydrophthalimid, Pentafluorbenzylalkohol, 4-Phenyl-3-chlor-2-buten-1-ol, 3-Trifluormethoxybenzylalkohol, 3-Dichlorvinyloxybenzylalkohol, 3-Propargyloxybenzylalkohol, 3-Dichlorvinyloxy-α-cyano-benzylalkohol, 3-Phenoxybenzylalkohol, 3-Phenyloxy-α-cyano-benzylalkohol, 3-Phenoxy-α-methoxycarbonyl-benzylalkohol, 3-Phenoxy-α-äthinyl-benzylalkohol, 3-Phenoxy-4-fluorbenzylalkohol, 3-(4'-Fluorphenoxy)-benzylalkohol, 3-(4'-Chlorphenoxy)-benzylalkohol, 3-(4'-Bromphenoxy)-benzylalkohol.

Als Verdünnungsmittel in dem Verfahrensschritt b kommen in erster Linie Wasser und Alkohole in Frage, wie Methanol, Ethanol, n-oder iso-Propanol, Butanol, tert.-Butanol, Glykol, Glykolmonomethylether oder die zugehörigen Alkohole der eingesetzten Alkoholate. Ferner sind Kohlenwasserstoffe wie Benzol, Toluol, chlorierte Kohlenwasserstoffe wie Methylenchlorid, Chlorbenzol, Ether wie THF, Dioxan und Dimethoxyethan oder polare Solventien wie DMF, DMSO, HMPT geeignet. Auch Zweiphasensysteme wie Toluol/Wasser oder Methylenchlorid/Wasser in Kombination mit üblichen Ammonium-, Phosphoniumsalzen oder Kronenethern als Phasentransferkatalysatoren kommen in Frage.

Die Reaktionstemperaturen können in einem größeren Bereich variiert werden. Im allgemeinen arbeitet man zwischen 20° und 180 °C, vorzugsweise zwischen 50 und 120 °C.

Die Umsetzung wird bei Normaldruck, kann aber auch unter Druck ausgeführt werden.

Bei der Durchführung des erfindungsgemäßen Verfahrens (Teil b) setzt man auf 1 Mol Addukt der Formeln IV oder V 3 bis 6 Äquivalente an Basen der Formel VI ein. Auch ein größerer Überschuß an Base ist möglich.

Die folgenden Beispiele illustrieren das erfindungsgemäße Verfahren.

Herstellung von 1-Chlor-3,3-dimethyl-pent-4-en-2-on (Verbindung III)

## Beispiel 1

a) 1-Chlor-3,3-dimethyl-2-phenoxy-1,4-pentadien

116 g (1 Mol) Natriumphenolat und 82,5 g (0,5 Mol) 1,1-Dichlor-3,3-dimethyl-1,4-pentadien werden in 500 ml DMF 8 Stunden unter Rückfluß erhitzt. Die Lösung wird mit Methylenchlorid verdünnt und mit verdünnter Natronlauge ausgeschüttelt. Nach Trocknen der $CH_2Cl_2$-Phase über $Na_2SO_4$ wird das Lösungsmittel im Vakuum abgezogen. Es bleiben 108,5 g (0,488 Mol, 97 %) Rohprodukt zurück, das destilliert wird. Bei $Kp._{0,5}$ 80-90 °C gehen 93,6 g (0,42 Mol, 84 %) 1-Chlor-3,3-dimethyl-2-phenoxy-1,4-pentadien über.

NMR ($CDCl_3$) : δ 1,25 (s, 6 H), 4,9-6,2 (—$CH=CH_2$), 5,85 (s, 1 H), 6,8-7.4 (m, 5 H).

b) 1-Chlor-3,3-dimethyl-pent-4-en-2-on

57 g (0,256 Mol) 1-Chlor-3,3-dimethyl-2-phenoxy-1,4-pentadien werden im Gemisch aus 250 ml Ameisensäure und 50 ml konzentrierter Salzsäure 1 Stunde auf 40° erwärmt. Dann wird mit 400 ml $CH_2Cl_2$ und Eis verdünnt und 3 x mit 2 n Natronlauge ausgeschüttelt. Nach dem Trocknen der Methylenchloridphase über $Na_2SO_4$ wird das Lösungsmittel abrotiert. Es bleiben 1-Chlor-3,3-dimethyl-pent-4-en-2-on zurück.

NMR ($CDCl_3$) : δ 1,3 (s, 6 H), 4,35 (s, 2 H), 5,0-6,2 (—$CH=CH_2$).

Addition von Tetrachlorkohlenstoff an Verbindung III.

Beispiel 2

14,65 g (0,1 Mol) 1-Chlor-3,3-dimethyl-pent-4-en-2-on (Verbindung III) werden unter Stickstoff in 50 ml Tetrachlormethan in Gegenwart von 0,9 g (0,001 Mol) Tris-(triphenylphosphin)-rutheniumdichlorid 25 Stunden unter Rückfluß erhitzt. Nach Filtration und Abziehen des Lösungsmittels im Vakuum wird an der Ölpumpe destilliert. Bei $Kp._{0,5}$ 130-140° isoliert man 24,0 g (80 mmol, 80 %) Adduktgemisch aus 40 % 1,4,6,6,6-Pentachlor-3,3-dimethylhexanon-2 (Verbindung IV) und 60 % 1,4-Dichlor-4-methyl-3-(2,2,2-trichlorethyl)-pentanon-2 (Verbindung V).

IV : NMR ($CDCl_3$) : δ 1,3 (s, 3 H), 1,35 (s, 3 H) 3,1 (m, 2 H) 4,4-4,5 (m, 3 H)

V : NMR ($CDCl_3$) : δ 1,6 (s, 3 H), 1,65 (s, 3 H), 2,8-3,8 (m, 3 H) 4,45 (AB, 2 H).

Beispiel 3

14,65 g (0,1 Mol) 1-Chlor-3,3-dimethyl-pent-4-en-2-on (Verbindung III) werden mit 47 g (0,3 Mol) Tetrachlormethan in 50 ml DMF in Gegenwart von 1,3 g $FeCl_3$ 6 $H_2O$, 1,1 g Benzoin und 0,4 g Dimethylaminhydrochlorid unter Stickstoff 12 Stunden auf 120 °C erhitzt. Man verdünnt mit Methylenchlorid und schüttelt mit Wasser mehrfach aus. Die Destillation liefert bei $Kp._{0,6}$ 133-145 °C 25,6 g (85 mmol, 85 %) Adduktgemisch, das ca. 90 % an V und 10 % an IV enthält.

Herstellung von 2,2-Dimethyl-3-(2,2-dichlorvinyl)-cyclopropan-carbonsäure und Ester

Beispiel 4

6,0 g (20 mmol) des Adduktgemisches der Verbindungen IV und V (Beispiel 3) werden mit 4 g (0,1 Mol) Natriumhydroxid in 40 ml Wasser 6 Stunden unter Rückfluß erhitzt. Die klare wäßrige Lösung wird durch Ausschütteln mit Methylenchlorid, das verworfen wird, gereinigt. Dann wird mit Salzsäure angesäuert und mit $CH_2Cl_2$ extrahiert. Aus der Methylenchloridlösung erhält man nach Trocknen und Abziehen des Lösungsmittels 4,1 g (19,6 mmol) 2,2-Dimethyl-3-(2,2-dichlorvinyl)-cyclopropancarbonsäure (Permethrinsäure) als cis-trans-Gemisch (GC : 43 % cis-Säure und 38,3 % trans-Säure).

NMR ($CDCl_3$) : δ 1,2-1,4 (m, 6 H), 1,5-2,4 (m, 2 H), 5,65 und 6,25 (2 d, 1 H), 10,6 (s, 1 H).

Beispiel 5

30 g (0,1 Mol) des Adduktgemisches der Verbindungen IV und V (Beispiel 3) werden mit 22,5 g (0,4 Mol) KOH in 300 ml Wasser 5 Stunden unter Rückfluß erhitzt. Die Aufarbeitung gemäß 4 ergibt 17,7 g (85 mmol, 85 %) 2,2-Dimethyl-3-(2,2-dichlorvinyl)-cyclopropancarbonsäure (Permethrinsäure) 50,2 % cis- und 41,0 % trans-Säure).

Beispiel 6

3,9 g (13 mmol) des Adduktgemisches der Verbindungen IV und V werden mit 2,6 g (65 mmol) NaOH in 30 ml Ethanol 5 Stunden unter Rückfluß erhitzt. Die Aufarbeitung mit Methylenchlorid und verdünnter Salzsäure führt zu 2,35 g (11,3 mmol, 87 %) Permethrinsäure als cis-trans-Gemisch.

Beispiel 7

In die Lösung von 2,3 g (0,1 Mol) Natrium in 100 ml abs. Ethanol gibt man 6 g (20 mmol), des Adduktgemisches der Verbindungen IV und V und erhitzt 8 Stunden unter Rückfluß. Man säuert an und schüttelt mit $CH_2Cl_2$ aus. Die Destillation bei $Kp._{0,15}$ 94-98 °C liefert 3,7 g (15,6 mmol, 78 %) cis- und trans-Permethrinsäureethylester.

2,2-Dimethyl-3-(2-chlor-3,3,3-trifluor-1-propenyl)-cyclopropancarbonsäure

Beispiel 8

14,56 g (0,1 mol) 1-Chlor-3,3-dimethyl-pent-4-en-2-on (III) werden mit 10,45 g (0,05 mol) 1,1,1-Trichlortrifluorethan in 50 ml DMF in Gegenwart von 1,3 g $FeCl_3$ 6 $H_2O$, 1,1 g Benzoin und 0,4 g Dimethylaminhydrochlorid unter Stickstoff 10 Stunden auf 120 °C erhitzt. Dann wird mit Methylenchlorid und Wasser aufgearbeitet. Nach Abziehen des getrockneten Lösungsmittels bleiben 22 g Rohprodukt zurück, das fraktioniert wird. Das Adduktgemisch ($Kp._{20}$ 130-150°) enthält 3-(2-Chlor-2-propyl)-1,5,5-trichlor-6,6,6-trifluorhexanon-2 als Hauptprodukt.

NMR ($CDCl_3$) : δ 1,65 (s, 6 H), 2,4-3,9 (m, 3 H), 4,6 (s, 2 H)

Beispiel 9

7,1 g (20 mmol) des Adduktes aus Beispiel 8 werden mit 4 g (0,1 mol) NaOH in 40 ml Wasser 6 Stunden unter Rückfluß erhitzt. Die Aufarbeitung gemäß Beispiel 4 führt zu 4,7 g (19,4 mmol, 97 %) 2,2-Dimethyl-3-(2-chlor-3,3,3-trifluor-1-propenyl)-cyclopropancarbonsäure als Isomerengemisch von Fp. 67-77°.

2,2-Dimethyl-3(-2,2-dibromvinyl)-cyclopropancarbonsäure

Beispiel 10

14,65 g (0,1 mol) 4-Chlor-3,3-dimethyl-pent-4-en-2-on (III) und 33,2 (0,1 mol) Tetrabrommethan werden 35 Stunden auf 90° erhitzt, wobei ca. 1 g Dibenzoylperoxid sukzessiv zugesetzt werden. Die Aufarbeitung mit $CH_2Cl_2$ und Wasser führt zu 47 g Rohprodukt, das fraktioniert wird. Das Adduktgemisch aus 1-Chlor-4-brom-4-methyl-3-(2,2,2-tribomethyl)-pentanon-2 und 1-Chlor-3,3-dimethyl-4,6,6,6-Tetrabrom-hexanon-2 wird bei $Kp._{0,09}$ 160-200° abgetrennt.

Beispiel 11

9,56 g (20 mmol) des Adduktgemisches aus Beispiel 10 werden mit 4 g (0,1 mol) NaOH in 80 ml Wasser 4 Stunden bei R. T. und 1 Stunde bei 80° gerührt. Die Aufarbeitung gemäß Beispiel 4 liefert 4,94 g (16,6 mmol, 83 %) 2,2-Dimethyl-3-(2,2-dibromvinyl)-cyclopropancarbonsäure als Isomeren gemisch, in dem die cis-Verbindung überwiegt.

NMR ($CDCl_3$) : δ 1,25-1,35 (4 s, 6 H), 1,6-2,2 (m, 2 H), 6,2 und 6,75 (d, 1 H).

**Ansprüche**

1. Verfahren zur Herstellung von 2,2-Dimethyl-3-vinyl-cyclopropancarbonsäure-Derivaten der Formel I

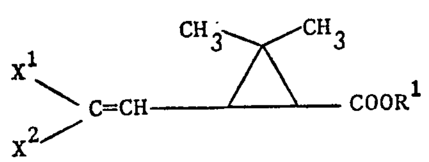

(I)

in welcher

$R^1$ für Wasserstoff, Alkyl oder den Rest eines bei Pyrethroiden verwendbaren Alkohols, steht und

$X^1$ und $X^2$ gleich oder verschieden sind und für Halogen oder fluorsubstituiertes Alkyl stehen, dadurch gekennzeichnet, daß man Polyhalogenalkan der Formel II

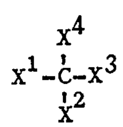

(II)

in welcher

$X^1$ und $X^2$ für die oben genannten Reste und

$X^3$ und $X^4$ gleich oder verschieden sind und für Halogen stehen an 1-Chlor-3,3-dimethyl-pent-4-en-2-on der Formel III

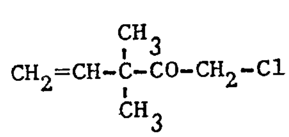

(III)

in Gegenwart von Katalysatoren die freie Radikale liefern, oder in Gegenwart von Metallsalzen der VIII Hauptgruppe oder der Nebengruppe IVa, VIIa und Ib des Periodensystems addiert und die dabei erhältlichen Verbindungen der Formeln IV und V

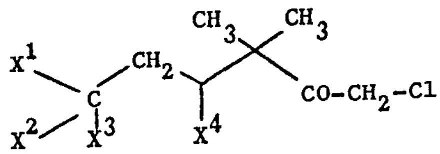

(IV)

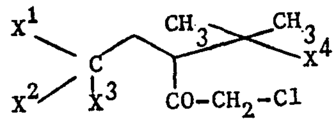

(V)

in welchen

$X^1$, $X^2$, $X^3$ und $X^4$ für die oben genannten Reste stehen einzeln oder im Gemisch mit Basen der Formel VI

$$(R^1 - O^{\ominus})_n\ M^{n+} \qquad \text{(VI)}$$

in welcher

$R^1$ für die oben genannten Reste,

M für Alkali- oder Erdalkalimetall und

n für 1 oder 2 stehen, umsetzt.

2. Verbindungen der Formel IV

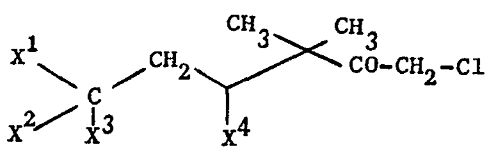

(IV)

in welchen

$X^1$ und $X^2$ gleich oder verschieden sind und für Halogen oder fluorsubstituiertes Alkyl

$X^3$ und $X^4$ gleich oder verschieden sind und für Halogen stehen.

3. Verbindungen der Formel V

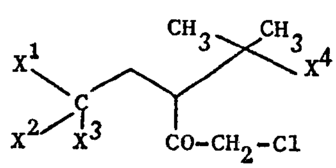

(V)

in welchen

$X^1$ und $X^2$ gleich oder verschieden sind und für Halogen oder fluorsubstituiertes Alkyl

$X^3$ und $X^4$ gleich oder verschieden sind und für Halogen stehen.

4. 1,4,6,6,6-Pentachlor-3,3-dimethyl-hexanon-2.

5. 1,4-Dichlor-4-methyl-3-(2,2,2-trichlorethyl)-pentanon-2.

**Claims**

1. Process for the preparation of 2,2-dimethyl-3-vinylcyclopropanecarboxylic acid derivatives of the formula I

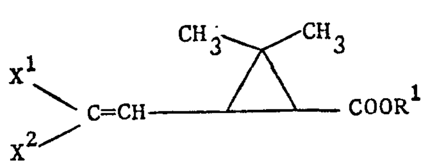

(I)

in which

$R^1$ represents hydrogen, alkyl or the radical of an alcohol which can be used in pyrethroids, and

$X^1$ and $X^2$ are identical or different and represent halogen or fluorinesubstituted alkyl, characterised in that a polyhalogenoalkane of the formula II

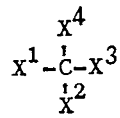

(II)

in which

$X^1$ and $X^2$ represent the abovementioned radicals and

$X^3$ and $X^4$ are identical or different and represent halogen, is added to 1-chloro-3,3-dimethyl-pent-4-en-2-one of the formula III

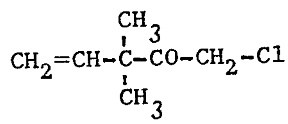

(III)

in the presence of catalysts which yield free radicals, or in the presence of metal salts of the VIII main group or the sub-group IVa, VIIa and Ib of the periodic system, and the compounds which are obtained thereby and are of the formulae IV and V

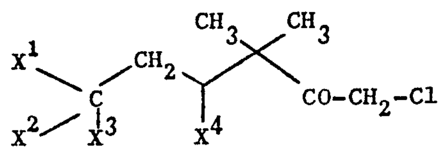

(IV)

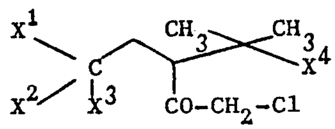

(V)

in which

$X^1$, $X^2$, $X^3$ and $X^4$ represent the abovementioned radicals, are reacted, individually or as a mixture, with bases of the formula VI

$$(R^1 - O^{\ominus})_n\ M^{n+} \qquad \text{(VI)}$$

in which

$R^1$ represents the abovementioned radicals,

M represents an alkali metal or alkaline earth metal, and

n represents 1 or 2.

2. Compounds of the formula IV

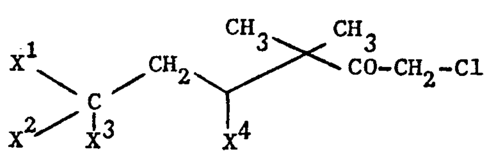

(IV)

in which

$X^1$ and $X^2$ are identical or different and represent halogen of fluorine-substituted alkyl, and

$X^3$ and $X^4$ are identical or different and represent halogen.

3. Compounds of the formula V

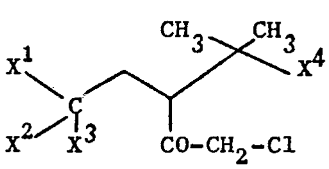

(V)

in which

$X^1$ and $X^2$ are identical or different and represent halogen or fluorine-substituted alkyl, and

$X^3$ and $X^4$ are identical of different and represent halogen.
4. 1,4,6,6,6-Pentachloro-3,3-dimethyl-hexan-2-one.
5. 1,4-Dichloro-4-methyl-3-(2,2,2-trichloroethyl)-pentan-2-one.

**Revendications**

1. Procédé de préparation de dérivés d'acides 2,2-diméthyl-3-vinyl-cyclopropane-carboxyliques de formule I

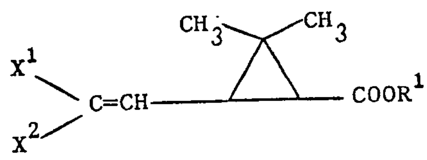 (I)

dans laquelle
$R^1$ représente un atome d'hydrogène, un groupe alkyle ou le radical d'un alcool pouvant être utilisé pour les pyréthroïdes, et
$X^1$ et $X^2$ sont identiques ou différents et représentent chacun un atome d'halogène ou un groupe alkyle substitué par un atome de fluor, caractérisé en ce qu'on ajoute un polyhalogéno-alcane de formule II

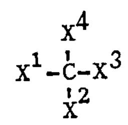 (II)

dans laquelle
$X^1$ et $X^2$ représentent les radicaux mentionnés ci-dessus, et
$X^3$ et $X^4$ sont identiques ou différents et représentent chacun un atome d'halogène, à la 1-chloro-3,3-diméthyl-pent-4-én-2-one de formule III

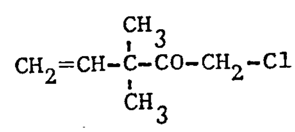 (III)

en présence de catalyseurs fournissant des radicaux libres, ou en présence de sels des métaux du groupe principal VIII ou des groupes IVa, VIIa et Ib du Système Périodique, et on fait réagir les composés ainsi obtenus répondant aux formules IV et V

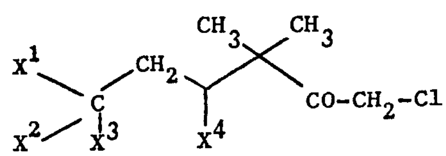 (IV)

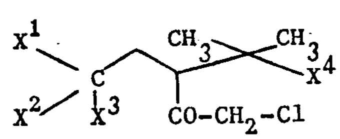 (V)

dans lesquelles
$X^1$, $X^2$, $X^3$ et $X^4$ représentent les radicaux mentionnés ci-dessus, individuellement ou en mélange, avec des bases de formule VI

$$(R' - O^{\ominus})_n \; M^{n+} \qquad \text{(VI)}$$

dans laquelle
$R^1$ représente les radicaux mentionnés ci-dessus,
M représente un métal alcalin ou un métal alcalino-terreux, et
n représente 1 ou 2.

2. Composés de formule IV

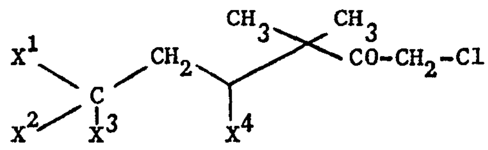

(IV)

dans laquelle

$X^1$ et $X^2$ sont identiques ou différents et représentent chacun un atome d'halogène ou un groupe alkyle substitué par un atome de fluor,

$X^3$ et $X^4$ sont identiques ou différents et représentent chacun un atome d'halogène.

3. Composés de formule V

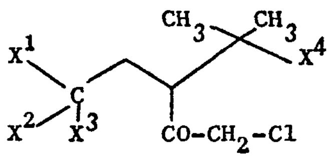

(V)

dans laquelle

$X^1$ et $X^2$ sont identiques ou différents et représentent chacun un atome d'halogène ou un groupe alkyle substitué par un atome de fluor,

$X^3$ et $X^4$ sont identiques ou différents et représentent chacun un atome d'halogène.

4. La 1,4,6,6,6-pentachloro-3,3-diméthylhexanone-2.

5. La 1,4-dichloro-4-méthyl-3-(2,2,2-trichloréthyl)-pentanone-2.